# EUROPÄISCHE PATENTSCHRIFT

(11) **EP 0 717 038 B1**
(45) Veröffentlichungstag und Bekanntmachung des Hinweises auf die Patenterteilung: **18.11.1998**
(21) Anmeldenummer: 95113519.3
(22) Anmeldetag: 29.08.1995
(51) Int. Cl.: C07D 239/42

(54) **Verfahren zur Herstellung von 2-Anilino-pyrimidin-Derivaten**
Process for the preparation of 2-anilino-pyramidine derivatives
Procédé pour la préparation de dérivés de 2-anilino-pyrimidine

(30) Priorität: 16.12.1994 DE 4444928
(43) Veröffentlichungstag der Anmeldung: 19.06.1996
(73) Patentinhaber: Hoechst Schering AgrEvo GmbH, 13509 Berlin (DE)
(72) Erfinder: Ressel, Hans-Joachim, Dl., D-65795 Hattersheim (DE); Schlegel, Günter, Dr., D-65835 Liederbach (DE)

(56) Entgegenhaltungen:
- EP-A- 0 295 210
- EP-A- 0 310 550
- J. HETEROCYCLIC CHEM., Bd. 22, 1985 Seiten 101-3, A. KREUTZBERGER 'Antimykotische Wirkstoffe. XX [1,2,3]. Fluorierte 2-(4-Toluidino)pyrimidine'

## Beschreibung

Die Erfindung betrifft ein Verfahren zur Herstellung substituierter 2-Anilino-pyrimidine der Formel I, 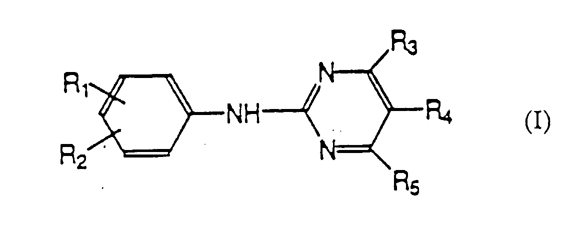
worin R₁ und R₂ unabhängig voneinander gleich oder verschieden Wasserstoff, Halogen, CN, C₁-C₃-Alkyl, C₁-C₃-Haloalkyl, C₁-C₃-Alkoxy, C₁-C₃-Haloalkoxy, Aryl und Aryloxy sind, wobei Alkyl- und Alkoxygruppen sowie deren halogensubstituierte Abkömmlinge linear oder verzweigt sein können;
   und
R₃, R₄ und R₅ unabhängig voneinander gleich oder verschieden
   Wasserstoff,
   Halogen,
   C₁-C₈-Alkyl,
   C₁-C₆-Alkyl substituiert durch Halogen oder Nitril,
   C₂-C₇-Alkenyl oder oder-Alkinyl, die Halogensubstituenten tragen können,
   C₃-C₇-Cycloalkyl, die durch Methyl und/oder Halogen bis zu dreifach gleich oder verschieden substituiert sein können,
   C₁-C₅-Mono- und Dialkylamino,
   C₁-C₅-Alkylcarbonyl-C₁-C₃-alkyl
   oder
   -(CH₂)ₙ-X-R₆ bedeuten, worin
   n eine positive ganze Zahl zwischen 1 und 6 ist,
   X für O oder S steht und
R₆ für
   C₁-C₈-Alkyl,
   C₂-C₅-Alkenyl oder Alkinyl,
   mit Halogen und/oder Nitrilo, C₁-C₄-Alkoxyalkoxy, C₁-C₄-Alkoxy,
   C₁-C₃-Alkylthio substituiertes C₁-C₆-Alkyl,
   C₂-C₆-Alkenyl oder Alkinyl,
   C₃-C₇-Cycloalkyl
   oder
   durch Methyl und/oder Halogen substituiertes C₃-C₇-Cyloalkyl steht,
   durch Umsetzung von Phenylguanidinsalzen der allgemeinen Formel II 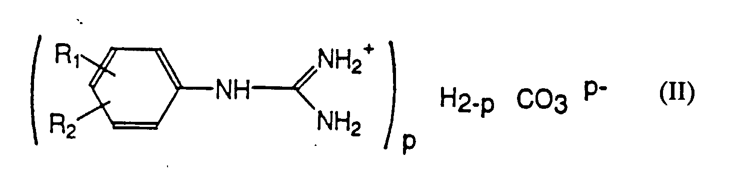
worin R₁ und R₂ die bei Formel I angegebene Bedeutung besitzen und p 1 oder 2 ist, mit 1,3-Diketonen der Formel III 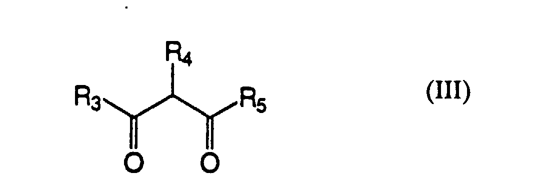
worin R₃, R₄ und R₅ die bei Formel I angegebene Bedeutung haben.

2-Anilino-pyrimidine sind bereits bekannt. Sie haben u.a. große Bedeutung als Zwischenprodukte und Wirkstoffe in Arznei- und Pflanzenschutzmitteln, u.a. als Fungizide. Auch Verfahren zu ihrer Herstellung sind bereits bekannt geworden. So ist beispielsweise die Synthese für die Reaktion der freien Phenylguanidine und der Phenylguanidin-hydrogencarbonate bzw. -carbonate mit 1,3-Diketonen beschrieben. In Anlehnung an eine von Kreutzberger, J. Het. chem., 22, 101 (1985) durchgeführte Methode erzielen EP 295210 und EP 310550 in der Schmelze der Hydrogencarbonat/1,3-Diketon-Mischungen oder mit einem Lösungsmittel Ausbeuten von 74 bis 92 % bei Durchführung der Reaktion unter Rückfluß bei Normaldruck. Gemäß dem in der Literatur beschriebenen Verfahren muß an die eigentliche Umsetzung in jedem Fall ein aufwendiges Reinigungsverfahren angeschlossen werden, um die angestrebten 2-Anilino-pyrimidin-Verbindungen in ausreichender Reinheit zu erhalten.

Es sind auch andere Herstellungsmethoden für 2-Anilino-pyrimidin-Derivate beschrieben worden, doch werden die Zielverbindungen entweder in niedrigen Ausbeuten erhalten oder es werden teure Reagenzien benötigt; z.B. Umsetzung von 2-Chlor-Pyrimidinen mit Anilinen (DD 151404, Bsp. 3) oder (EP 224339) Umsetzung von Formaniliden mit 2-Methansulfonylpyrimidinen.

Angesichts des hierin genannten und diskutierten Standes der Technik war es Aufgabe der Erfindung, ein Verfahren zur Herstellung von 2-Anilino-pyrimidin-Derivaten anzugeben, das die Verwendung teurer Chemikalien weitgehendst vermeidet und gleichzeitig die erwünschten Produkte in besserer Ausbeute zugänglich macht, als dies mit den Verfahren gemäß dem Stand der Technik möglich war.

Gelöst werden diese und weiteren nicht näher aufgeführte Aufgaben durch ein Verfahren der eingangs erwähnten Gattung mit den Merkmalen des kennzeichnenden Teils des Anspruches 1. Vorteilhafte Verfahrensmodifikationen werden in den auf diesen Anspruch rückbezogenen abhängigen Ansprüchen unter Schutz gestellt.

Dadurch, daß die Umsetzung des Phenylguanidinsalzes der allgemeinen Formel II mit dem 1,3-Diketon der allgemeinen Formel III unter vermindertem Druck und unter Abtrennung des entstehenden Reaktionswassers sowie des entstehenden Kohlendioxids durchgeführt wird, gelangt man in nicht vorab zu übersehender Weise zu einem Verfahren, das sich durch annähernd quantitative Ausbeuten und sehr hohe Reinheit des Produktes sowie zusätzlich durch eine äußerst geringe Belastung der Umwelt durch zu entsorgende Stoffe auszeichnet.

Erfindungsgemäß hat sich dabei sowohl der Einsatz des Phenylguanidinhydrogencarbonats der Formel IIa 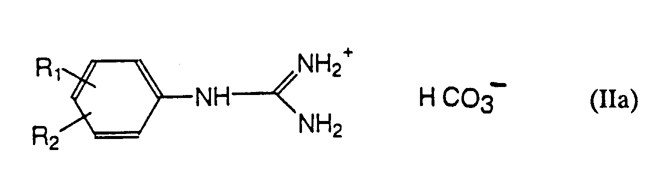 als auch des Phenylguanidincarbonats der Formel IIb 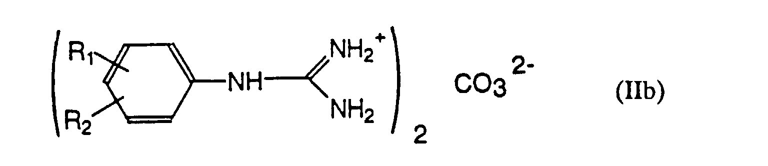 bewährt. Auch die Verwendung eines Gemisches dieser beiden Salze ist im Rahmen der Erfindung möglich.

Im einzelnen führt die Reaktion von Phenylguanidinhydrogencarbonat und Phenylguanidincarbonat mit 1,3-Diketonen unter vermindertem Druck und unter Auskreisen des entstehenden Wassers im Vergleich zu den bekannten Herstellungsmethoden überraschenderweise zu folgenden deutlichen Vorteilen:
a) Durch die Entfernung des Reaktionswassers und des während der Reaktion freigesetzten Kohlendioxids werden Nebenreaktionen ausgeschlossen, die durch verbleibendes Wasser ausgelöst werden können.
b) Durch Arbeiten unter vermindertem Druck wird die thermische Belastung der Zielprodukte gering gehalten.
c) Es werden fast quantitative Ausbeuten der 2-Anilino-pyrimidine (Formel I) erhalten, wobei die Reinheit der Verbindungen sehr hoch ist und eine weitere Aufreinigung unnötig ist.
d) Die Phenylguanidinsalze der Formel IIa und IIb können als wasserfeuchte Ware eingesetzt werden, so daß eine vorherige Trocknung vorteilhafter Weise entfallen kann.
e) Das 1,3-Diketon (Formel III) ist unter den Reaktionsbedingungen (niedrige Temperaturen durch verminderten Druck und Entfernen des Wassers) stabil; der ggf. verwendete Überschuß kann praktisch quantitativ zurückgewonnen und wiederverwendet werden. Bei der Reaktion fallen daher nur eine Wasserphase und Kohlendioxid als Abgangsstoffe an.

Unter "vermindertem Druck" wird erfindungsgemäß ein Druck unterhalb des Atmosphärendrucks von 1013 hPa verstanden. Das erfindungsgemäße Verfahren läßt sich ohne weiteres in einem weiten Bereich unterhalb des Atmosphärendrucks durchführen, wobei es sich versteht, daß der Druck während der Reaktion nicht ständig sondern lediglich überwiegend im für die Erfindung günstigen Bereich sein soll.

In bevorzugter Verfahrensabwandlung wird der Druck während der Reaktion im Bereich zwichen 10 und 900 hPa gehalten.

Die Abtrennung des während der Reaktion entstehenden Wassers kann grundsätzlich auf jede dem Fachmann geläufige und zur Entfernung von Wasser aus einem Reaktionsgemisch geeignete Weise durchgeführt werden. Von Vorteil ist es beispielsweise, das Reaktionswasser abzudestilllieren. Hierfür geeignete Apparaturen sind dem Fachmann bekannt.

In besonders günstiger Modifikation wird das Verfahren der Erfindung so ausgeführt, daß der Druck im Verlauf der Reaktion graduell oder kontinuierlich verringert wird, während die Temperaturen der Reaktionsmischung durch Wärmezufuhr gleichzeitig graduell oder kontinuierlich in solch einem Maße gesteigert wird, daß ein stetiges oder kontinuierliches Abscheiden des Wassers vorzugsweise in einem Kühlerkondensat gewährleistet wird.

Dabei ist es weiterhin bevorzugt, das Wechselspiel von Temperaturerhöhung und Druckerniedrigung so zu betreiben, daß sich die zu Beginn der Umsetzung inhomogene Reaktionsmischung aus Phenylguanidinsalz und 1,3-Diketon im Verlauf der Reaktion mit fortschreitender Umsetzung in eine klare Flüssigphase umwandelt.

Dabei kann die anzunehmende Temperatur über einem weiten Bereich variieren. Sie liegt jedoch bevorzugt je nach Edukten zwischen 40 und 120°C, vorzugsweise bei 55 bis 95°C.

In der Erfindung wird das 1,3-Diketon (Formel III) in 1,0 bis 3-facher Menge, vorzugsweise in 1,0 bis 1,5-facher molarer Menge, bezogen auf das Phenylguanidinsalz der Formel II eingesetzt. Es ist kein zusätzliches Lösungsmittel erforderlich, jedoch kann die Reaktion auch in der Schmelze des Produktes durchgeführt werden.

Für die Herstellung der Phenylguanidin-hydrogencarbonate (Formel IIa) und - carbonate (Formel IIb) sind in der Literatur Methoden beschrieben:
Firmenbroschüre der SKW Trostberg AG, S. 97 (1978), EP 560726, worauf aus Offenbarungsgründen Bezug genommen wird.

Vorzugsweise wird die Reaktion der Erfindung so durchgeführt, daß man das Phenylguanidin-hydrogencarbonat oder -carbonat und das 1,3-Diketon unter Rühren und Wasserabtrennung unter vermindertem Druck bei den gewünschten Temperaturen so lange zur Reaktion bringt, bis kein Wasser mehr abgeschieden wird. Anschließend werden Reste des 1,3-Diketons abdestilliert. Das erhaltene Produkt erstarrt beim Abkühlen. Es zeichnet sich durch seine hohe Reinheit aus.

Obwohl das Verfahren der Erfindung sich für die Umsetzung einer Vielzahl von Verbindungen der Formeln II und III vorteilhaft eignet, zeigt es doch besonders gute Ergebnisse, wenn
Verbindungen der Formel II, worin
R₁ und R₂ unabhängig voneinander gleich oder verschieden
   Wasserstoff,
   Halogen und
   Nitril sind,
   mit
   Verbindungen der Formel III gemäß Anspruch 1,
   worin
R₄ Wasserstoff ist und
R₃ und R₅ unabhängig voneinander gleich oder verschieden
   C₁-C₄-Alkyl,
   C₃-C₇-Cycloalkyl,
   -(CH₂)ₙ-O-R₆ mit n = 1 oder 2 und
R₆ = C₁-C₈-Alkyl
   oder
   C₂-C₇-Alkenyl oder Alkinyl sind, umgesetzt werden.

Ganz besonders vorteilhaft verläuft die erfindungsgemäße Umsetzung, wenn Phenylguanidincarbonat oder -hydrogencarbonat (R₁ = R₂ = H) mit Acetylaceton (R₄ = H, R₃ = R₅ = CH₃) umgesetzt wird.

Die folgenden Beispiele dienen zur Erläuterung der Erfindung:

### Beispiel 1: 2-Anilino-4,6-dimethylpyrimidin

Phenylguanidin-hydrogencarbonat (50 g; 99 %ig) und Acetylaceton (28,7 g, 99,5 %ig) werden unter Rühren in einem Rundkolben mit angeschlossenem Dean-Stark-Wasserabscheider und Rückflußkühler bei einem konstanten Vakuum von 260 hPa langsam erhitzt.

Bei etwa 55°C beginnt die Abscheidung einer wässrigen Phase und es kommt zur CO₂-Entwicklung. Die Temperatur wird innerhalb von 2 Stunden auf 75°C erhöht unter gleichzeitigem Absenken des Druckes auf 190 hPa. Während dieser Zeit scheiden sich insgesamt 15,5 ml der wässrigen Phase ab. Das anfangs inhomogene Reaktionsgemisch wandelt sich in eine orange-braune, klare Flüssigphase um.

Jetzt wird unter Temperaturerhöhung auf 95°C und Druckminderung bis 100 hPa noch für eine Stunde gerührt. Anschließend wird das verbliebene Acetylaceton durch Absenken des Innendruckes auf zuletzt 10 hPa komplett abdestilliert. Das Produkt wird in eine flache Schale gegossen, wo es beim Abkühlen zu beigefarbenen Kristallen erstarrt.

Ausbeute: 50,5 g (98,9 %ig HPLC = 99,8 % d.Th.)

### Beispiel 2: 2-Anilino-4,6-dimethylpyrimidin

In einen Edelstahl-Mischreaktor mit angesetztem Dean-Stark-Wasserabscheider und effektiver Rührung werden Phenylguanidin-hydrogencarbonat (1170,5 g; 88 %ig wasserfeucht) und Acetylaceton (800 ml; 99,5 %ig) gefüllt. Es wird Vakuum (250 hPa) angelegt. Beginnend bei einer Temperatur von 55°C scheidet sich Wasser ab und es erfolgt Kohlendioxid-Entwicklung. Die Temperatur im Reaktor wird im Verlauf von 4 Stunden auf 80°C erhöht, wobei gleichzeitig der Druck bis 130 hPa reduziert wird. Die inhomogene Reaktionsmischung geht in eine bräunliche, klare Flüssigphase über.

Anschließend wird für 1 Stunde bei 80-82°C und 130 hPa gerührt. Dann wird durch langsames Reduzieren des Druckes bis 30 hPa der größte Teil des verbliebenen Acetylacetons abdestiliert.

Von der abgeschiedenen Wasserphase weden nun bei 280 hPa und 80°C 150 g zum Reaktionsgemisch zudosiert, wobei letzte Reste des Acetylacetons mit dem Wasser über den Wasserabscheider ausgeschleppt werden. Der Druck wird bei gleicher Temperatur langsam bis 15 hPa reduziert. Das Produkt beginnt bei ∼ 80°C unter Rühren zu kristallisieren und liegt nach langsamem Abkühlen auf Raumtemperatur in Form von gut austragbaren Kristallen vor.

Ausbeute: 1042,0 g (99,7 %ig HPLC = 99,8 % d.Th.).

Es werden insgesamt 516 g wässrige Phase (82 % H₂O/18 % Acetylaceton) und 505 g Acetylaceton-Phase (98 % Acetylaceton/2 % Wasser) abgetrennt.

Aus der wässrigen Phase kann durch Andestillieren das enthaltene Acetylaceton zurückgewonnen werden. Das zurückgewonnene Acetylaceton ist wieder einsetzbar.

### Beispiel 3: Herstellung von 2-Anilino-4,6-dimethylpyrimidin

Phenylguanidin-carbonat (170,6 g; 96,7 %ig) wird mit Acetylaceton (125,6 g; 99,5 %ig) analog Beispiel 1 umgesetzt.

Ausbeute: 203,5 g (95,5 %ig HPLC = 97,6 % d.Th.).

### Vergleichsbeispiel 4: 2-Anilino-4,6-dimethylpyrimidin (analog EP 295 210, Beispiel 1)

Verfahren wie unter Beispiel 1 beschrieben bei 100°C und gleicher Reaktionszeit, jedoch ohne Abdestillieren des Wassers.

Ausbeute: 49,9 g (84,2 %ig HPLC = 87,9 % d.Th.).

### Vergleichsbeispiel 5: 2-Anilino-4,6-dimethylpyrimidin

Verfahren analog Vergleichsbeispiel 4, jedoch bei 80°C.

Ausbeute: 51,8 g (85,2 %ig HPLC = 92,3 % d.Th.).

### Beispiel 6: 2-Anilino-4-methyl-6-cyclopropylpyrimidin

Phenylguanidin-hydrogencarbonat (10,0 g, 51 mmol) und 1-Cyclopropyl-1,3-butandion (9,7 g, 77 mmol) werden analog Beispiel 1 umgesetzt.

Ausbeute: 11,4 g (99,4 %ig HPLC = 99,4 d.Th.).

### Vergleichsbeispiel 7: 2-Anilino-4-methyl-6-cyclopropylpyrimidin (analog EP 310 550)

Phenylguanidin-hydrogencarbonat (10 g, 51 mmol) und 1-Cyclopropyl-1,3-butandion (9,7 g, 77 mmol) werden 6 Stunden unter Rühren auf 110°C erwärmt. Nach beendeter Kohlendioxidentwicklung und Abkühlen auf Raumtemperatur wird in Diethylether gelöst und 2 x mit Wasser gewaschen. Nach Trocknen über Natriumsulfat wird filtriert und das Lösungsmittel verdampft. Das Rohprodukt (12,9 g) wird über eine Kieselgel-Säule chromatographisch gereinigt. (LM: Toluol/Ethylacetat = 6/4). Es verbleiben nach Abdampfen des Lösungsmittels
9,2 g Produkt (94,2 %ig HPLC = 75,5 % d.Th.).

### Vergleichsbeispiel 8: (analog EP 310 550 Beispiel 1.2)

Phenylguanidin-hydrogencarbonat (118,2 g; 99 %ig) und Acetylaceton (71,2 g; 99,5 %ig) in 400 ml Ethanol werden unter Rühren 5 Stunden am Rückfluß erhitzt. Nach beendeter Kohlendioxid-Entwicklung wird auf Raumtemperatur abgekühlt und mit 800 ml Diethylether versetzt, 2 x mit je 300 ml Wasser gewaschen, über Natriumsulfat getrocknet, filtriert und eingedampft. Das verbleibende Rohprodukt wird über eine Kieselgel-Säule (Toluol/Ethylacetat = 5/2) gereinigt. Es werden nach Abdampfen der Lösungsmittel 99,1 g 2-Anilino-4,6-dimethylpyrimidin (99 %ig HPLC = 87,2 % d.Th.) erhalten.

### Vergleichsbeispiel 9:

Phenylguanidin, freie Base (335,7 g; 93,5 %ig), werden in iso-Propanol (320 ml) gelöst und auf 80°C (Rückfluß) erwärmt. Nun tropft man unter Rühren während 3 Stunden bei gleichen Bedingungen Acetylaceton (339,2 g; 99,5 %ig) zu. Man läßt noch für 2 Stunden nachrühren unter Rückfluß und kühlt dann unter Rühren auf + 5°C ab. Die ausgefallenen Kristalle werden abgesaugt und mit wenig iso-Propanol kalt gewaschen.

Nach Trocknung erhält man 349,4 g 2-Anilino-4,6-dimethylpyrimidin (96,1 %ig HPLC = 72,5 % d.Th.). Aus der Mutterlauge können noch 12,3 % d. Th. an 76,5 %igem Produkt gewonnen werden.

Weitere Vorteile und Ausführungsformen der Erfindung ergeben sich aus den nachfolgenden Patentansprüchen.

## Patentansprüche

1. Verfahren zur Herstellung von 2-Anilino-pyrimidin-Derivaten der allgemeinen Formel I 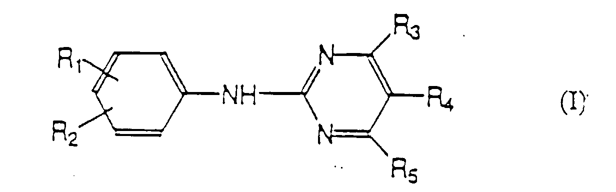
worin R₁ und R₂ unabhängig voneinander gleich oder verschieden Wasserstoff, Halogen, CN, C₁-C₃-Alkyl, C₁-C₃-Haloalkyl, C₁-C₃-Alkoxy, C₁-C₃-Haloalkoxy, Aryl und Aryloxy sind, wobei Alkyl- und Alkoxygruppen sowie deren halogensubstituierte Abkömmlinge linear oder verzweigt sein können;
und
R₃, R₄ und R₅ unabhängig voneinander gleich oder verschieden
Wasserstoff,
Halogen,
C₁-C₈-Alkyl,
C₁-C₆-Alkyl substituiert durch Halogen oder Nitril,
C₂-C₇-Alkenyl oder-Alkinyl, die Halogensubstituenten tragen können,
C₃-C₇-Cycloalkyl, die durch Methyl und/oder Halogen bis zu dreifach gleich oder verschieden substituiert sein können,
C₁-C₅-Mono- und Dialkylamino,
C₁-C₅-Alkylcarbonyl-C₁-C₃-alkyl
oder
-(CH₂)ₙ-X-R₆ bedeuten,
worin
n eine positive ganze Zahl zwischen 1 und 6 ist,
X für O oder S steht und
R₆ für
C₁-C₈-Alkyl,
C₂-C₅-Alkenyl oder-Alkinyl,
mit Halogen, Nitrilo, C₁-C₄-Alkoxyalkoxy, C₁-C₄-Alkoxy und/oder C₁-C₃-Alkylthio substituiertes C₁-C₆-Alkyl, C₂-C₆-Alkenyl oder -Alkinyl,
C₃-C₇-Cycloalkyl
oder
durch Methyl und/oder Halogen substituiertes C₃-C₇-Cycloalkyl steht,
durch Umsetzung von Phenylguanidinsalzen der allgemeinen Formel II 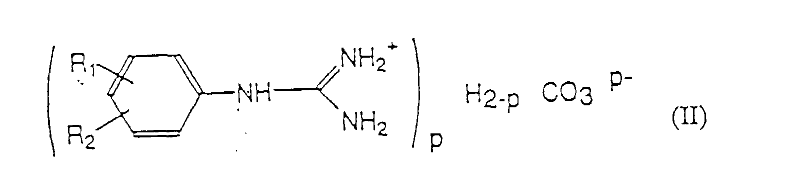
worin R₁ und R₂ die bei Formel I angegebenen Bedeutungen besitzen und p 1 oder 2 ist, mit 1,3-Diketonen der Formel III 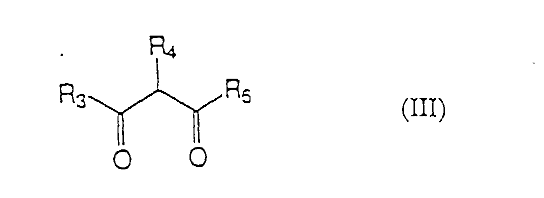
worin R₃, R₄ und R₅ die bei Formel I angegebene Bedeutung haben, dadurch gekennzeichnet, daß die Umsetzung unter einem Druck unterhalb von 1013 hPa und unter Abtrennung des entstehenden Wassers und Kohlendioxids durchgeführt wird.

2. Verfahren nach Anspruch 1, dadurch gekennzeichnet, daß der Druck zwischen 10 und 900 hPa gehalten wird.

3. Verfahren nach einem der Ansprüche 1 oder 2, dadurch gekennzeichnet, daß das während der Reaktion gebildete Wasser abdestilliert wird.

4. Verfahren nach einem der Ansprüche 1 bis 3, dadurch gekennzeichnet, daß der Druck graduell mit der im Verlauf der Reaktion langsam gesteigerten Temperatur so erniedrigt wird, daß ein kontinuierliches Abscheiden des Wassers im Kühlerkondensat gewährleistet ist.

5. Verfahren nach Anspruch 4, dadurch gekennzeichnet, daß sich die zu Beginn der Umsetzung inhomogene Reaktionsmischung in eine klare Flüssigphase umwandelt.

6. Verfahren nach einem der vorhergehenden Ansprüche, dadurch gekennzeichnet, daß die Reaktion bei einer Temperatur zwischen 40 und 120°C, vorzugsweise 55 und 95°C, ausgeführt wird.

7. Verfahren nach einem der vorhergehenden Ansprüche, dadurch gekennzeichnet, daß die Reaktion ohne Zusatz eines Lösungsmittels durchgeführt wird.

8. Verfahren nach einem der vorhergehenden Ansprüche, dadurch gekennzeichnet, daß Verbindungen der Formel II gemäß Anspruch 1, worin
R₁ und R₂ unabhängig voneinander gleich oder verschieden
Wasserstoff,
Halogen und
Nitril sind,
mit
Verbindungen der Formel III gemäß Anspruch 1,
worin
R₄ Wasserstoff ist und
R₃ und R₅ unabhängig voneinander gleich oder verschieden
C₁-C₄-Alkyl,
C₃-C₇-Cycloalkyl,
-(CH₂)ₙ-O-R₆ mit n = 1 oder 2 und
R₆ = C₁-C₈-Alkyl
oder
C₂-C₇-Alkenyl oder-Alkinyl sind,
umgesetzt werden.

9. Verfahren nach Anspruch 8, dadurch gekennzeichnet, daß Phenylguanidincarbonat oder -hydrogencarbonat (R₁ = R₂ = H) mit Acetylaceton (R₄ = H, R₃ = R₅ = CH₃) umgesetzt wird.

## Claims

1. A process for the preparation of 2-anilinopyrimidine derivatives of the formula I 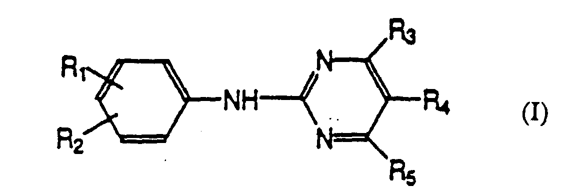
in which R₁ and R₂ independently of one another are identical or different and are hydrogen, halogen, CN, C₁-C₃-alkyl, C₁-C₃-haloalkyl, C₁-C₃-alkoxy, C₁-C₃-haloalkoxy, aryl and aryloxy, it being possible for alkyl and alkoxy groups and for their halogen-substituted derivatives to be linear or branched; and
R₃, R₄ and R₅ independently of one another are identical or different and are hydrogen,
halogen,
C₁-C₈-alkyl,
C₁-C₆-alkyl which is substituted by halogen or nitrile, C₂-C₇-alkenyl or -alkinyl, it being possible for these to be substituted by halogen,
C₃-C₇-cycloalkyl which can be up to trisubstituted by identical or different methyl and/or halogen substituents,
C₁-C₅-mono- and dialkylamino,
C₁-C₅-alkylcarbonyl-C₁-C₃-alkyl
or
-(CH₂)ₙ-X-R₆,
in which
n is a positive integer between 1 and 6,
X is O or S and
R₆ is
C₁-C₈-alkyl,
C₂-C₅-alkenyl or -alkinyl,
C₁-C₆-alkyl, C₂-C₆-alkenyl or -alkinyl which are substituted by halogen , nitrilo, C₁-C₄-alkoxyalkoxy, C₁-C₄-alkoxy and/or C₁-C₃-alkylthio, C₁-C₃-alkylthio,
C₃-C₇-cycloalkyl or C₃-C₇-cycloalkyl which is substituted by methyl and/or halogen by reacting phenylguanidine salts of the formula II 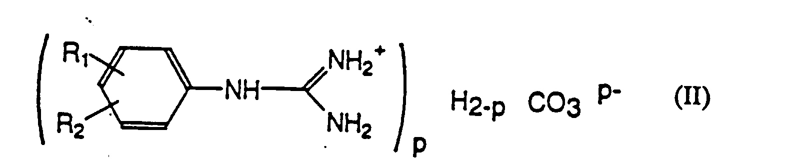
in which R₁ and R₂ have the meanings given for formula I and p is 1 or 2 with 1,3-diketones of the formula III 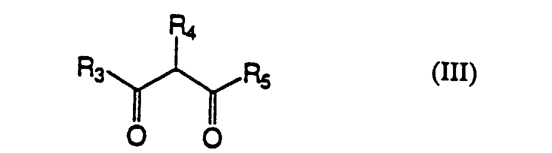
in which R₃, R₄ and R₅ have the meanings given for formula I,
which comprises the reaction being carried out under a pressure of below 1013 hPa and with removal of the resulting water and carbon dioxide.

2. The process as claimed in claim 1, wherein the pressure is kept between 10 and 900 hPa.

3. The process as claimed in one of claims 1 or 2, wherein the water formed during the reaction is distilled off.

4. The process as claimed in any of claims 1 to 3, wherein the pressure is reduced gradually as the temperature is raised slowly in the course of the reaction so that continuous separation of the water in the condenser condensate is guaranteed.

5. The process as claimed in claim 4, wherein the reaction mixture, which is inhomogenous at the beginning of the reaction, changes into a clear liquid phase.

6. The process as claimed in one of the preceding claims, wherein the reaction is carried out at a temperature between 40 and 120°C, preferably 55 and 95°C.

7. The process as claimed in one of the preceding claims, wherein the reaction is carried out without addition of a solvent.

8. The process as claimed in one of the preceding claims, wherein compounds of the formula II as set out in claim 1 in which R₁ and R₂ independently of one another are identical or different and are
hydrogen,
halogen and
nitrile
are reacted with compounds of the formula III as set
out in claim 1
in which
R₄ is hydrogen and
R₃ and R₅ independently of one another are identical or different and are
C₁-C₄-alkyl,
C₃-C₇-cycloalkyl
-(CH₂)ₙ-O-R₆ where n = 1 or 2 and
R₆ = C₁-C₈-alkyl or
C₂-C₇-alkenyl or -alkinyl.

9. The process as claimed in claim 8, wherein phenylguanidine carbonate or phenylguanidine hydrogen carbonate (R₁ = R₂ = H) is reacted with acetylacetone (R₄ = H, R₃ = R₅ = CH₃).

## Revendications

1. L'invention concerne un procédé de préparation de dérivés de 2-anilino-pyrimidine de formule générale I, 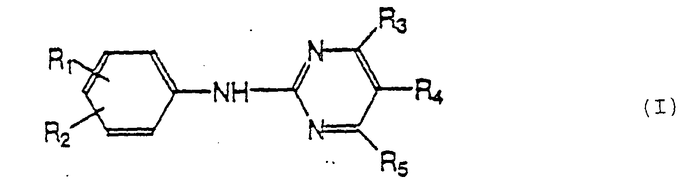
où R₁ et R₂ identiques ou différents, représentent indépendamment l'un de l'autre, un atome d'hydrogène, un atome d'halogène, des groupes -CN, alkyle en C₁-C₃, haloalkyle en C₁-C₃, alkoxy en C₁-C₃, haloalkoxy en C₁-C₃, aryle et aryloxy, les groupes alkyle et alkoxy, de même que leurs dérivés halogénés, pouvant être à chaîne linéaire ou ramifiée ;
et
R₃, R₄ et R₅, identiques ou différents, représentent, indépendamment l'un de l'autre,
un atome d'hydrogène,
un atome d'halogène,
un groupe alkyle en C₁-C₈,
un groupe alkyle en C₁-C₆ substitué par un atome d'halogène ou un groupe nitrile,
un groupe alcènyle ou alcynyle en C₂-C₇, qui peuvent porter la substitution par un atome d'halogène,
un groupe cycloalkyle en C₃-C₇ qui peut être substitué par un groupe méthyle et/ou un atome d'halogène jusqu'à trois fois par des substituants identiques ou différents,
un mono- et di(alkyl en C₁-C₅)amino,
(alkyl en C₁-C₅)carbonyl-alkyle en C₁-C₃ ou
- (CH₂)ₙ-X-R₆,
où
n est un nombre entier positif compris entre 1 et 6,
X représente un atome d'oxygène ou de soufre et
R₆ représente un groupe alkyle en C₁-C₈,
un groupe alcènyle ou alcynyle en C₂-C₅,
un groupe alkyle en C₁-C₆, un groupe alcènyle ou alcynyle en C₂-C₆,
substituè par un atome d'halogène un groupe nitrilo, des groupes alkoxyalkoxy en C₁-C₄ et/ou un groupe (alkyl en C₁-C₃) thio, un groupe cycloalkylene en C₃-C₇,
ou
un cycloalkyle en C₃-C₇ substitué par un groupe méthyle et/ou par un atome d'halogène,
par réaction de sels de phénylguanidine de formule générale II, 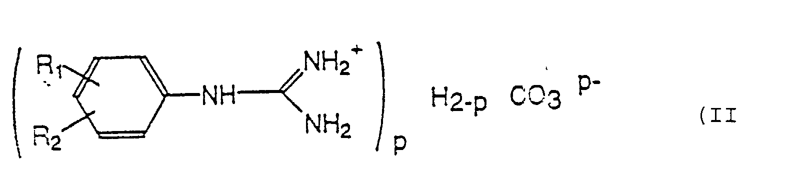
où R₁ et R₂ possèdent les significations données à la formule I et p vaut 1 ou 2, avec des 1,3-dicétones de formule III 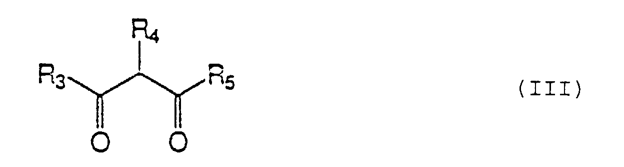
où R₃, R₄ et R₅ possèdent la signification donnée à la formule I, caractérisé en ce qu'on met en oeuvre la réaction sous une pression inférieure à 1013 hPa et en séparant l'eau et le dioxyde de carbone formés.

2. Procédé selon la revendication 1, caractérisé en ce que l'on maintient la pression entre 10 et 900 hPa.

3. Procédé selon l'une des revendications 1 ou 2, caractérisé en ce que l'on sépare par distillation l'eau formé au cours de la réaction.

4. Procédé selon l'une des revendications 1 à 3, caractérisé en ce que, au cours de la réaction, on diminue la pression progressivement, en fonction de la lente augmentation de la température de façon telle que l'on assure une séparation en continue de l'eau dans le condensat dans le réfrigérant.

5. Procédé selon la revendication 4, caractérisé en ce que le mélange réactionnel non homogène au début de la réaction, se transforme en une phase liquide limpide.

6. Procédé selon l'une des revendications précédentes, caractérisé en ce que l'on met en oeuvre la réaction à une température comprise entre 40 et 120 °C, de préférence entre 55 et 95 °C.

7. Procédé selon l'une des revendications précédentes, caractérisé en ce que l'on met en oeuvre la réaction sans ajout de solvant.

8. Procédé selon l'une des revendications précédentes, caractérisé en ce que l'on fait réagir les composés de formule II selon la revendication 1, où
R₁ et R₂, identiques ou différents, représentent, indépendamment l'un de l'autre,
un atome d'hydrogène,
un atome d'halogène et
un groupe nitrile,
avec des composés de formule III selon la revendication 1,
où
R₄ représente un atome d'hydrogène et
R₃ et R₅, identiques ou différents, représentent, indépendamment l'un de l'autre,
un groupe alkyle en C₁-C₄,
un groupe cycloalkyle en C₃-C₇,
-(CH₂)ₙOR₆ avec n = 1 ou 2 et
R₆ = alkyle en C₁-C₈
ou
un groupe alcényle ou alcynyle en C₂-C₇.

9. Procédé selon la revendication 8, caractérisé en ce que l'on fait réagir le carbonate ou le bicarbonate de phénylguanidine (R₁ = R₂ = H) avec l'acétylacétone (R₄ = H, R₃ = R₅ = CH₃).
